# EUROPEAN PATENT APPLICATION

(11) **EP 3 960 079 A1**
(43) Date of publication of application: **02.03.2022**
(21) Application number: 20193725.7
(22) Date of filing: 31.08.2020
(51) Int. Cl.: A61B 5/145, A61B 5/00

(54) **APPARATUS FOR INSERTING A MEDICAL DEVICE INTO A BODY TISSUE**

(71) Applicant: Roche Diabetes Care GmbH, 68305 Mannheim (DE); F. Hoffmann-La Roche AG, 4070 Basel (CH)
(72) Inventor: WIEGAND, Roland Hans, 68305 Mannheim (DE)
(74) Representative: Altmann Stößel Dick Patentanwälte PartG mbB

(57) **Abstract**

An apparatus (110), specifically a medical system (112), is disclosed, the apparatus (110) comprising:
a. a medical device (114) which is at least partially insertable into a body tissue of a user;
b. an insertion device (116) for at least partially inserting the medical device (114) into the body tissue;
c. a housing (118); and
d. a removable cap (120) connected to the housing (118), the removable cap (120) being configured for being removed from the housing (118) before insertion of the medical device (114),
wherein the housing (118) and the removable cap (120), prior to removal of the removable cap (120), are connected via at least one flexible connection element (122), wherein the flexible connection element (122) is reversibly displaceable from a locking position (216) in which the removable cap (120) is secured to the housing (118) by the flexible connection element (122) into a releasing position (218) in which the removable cap (120) is released, wherein the flexible connection element (122), prior to use, is secured in the locking position (216) by at least one frangible securing element (124).

Further, a method for preparing the apparatus (110) for insertion of at least a part of the medical device (114) into the body tissue of the user and a method for at least partially inserting at least a part of the medical device (114) of the apparatus (110) into the body tissue of the user are disclosed.

## Description

### Technical Field

This invention relates to an apparatus, specifically a medical system, and to methods for preparing the apparatus and for at least partially inserting at least a part of a medical device of the apparatus. The apparatus and methods may be applied, as an example, in the field of continuous monitoring of at least one analyte in a body fluid of a user. Additionally or alternatively, the apparatus and methods may be applied in the field of medication, such as in the field of infusion technology. The invention may be applied in the field of home care and/or in the field of professional care, such as in hospitals. Other applications are feasible.

### Background art

In medical technology and medicine, in various instances, medical devices have to be inserted into body tissue, such as transcutaneously and/or subcutaneously. As an example, analytical sensors, specifically for monitoring one or more body functions, often are to be inserted or implanted into body tissue. Further, certain medication devices such as infusion sets also, in many instances, require transcutaneous insertion of an infusion cannula. Other examples for insertion or implantation of medical devices may be given. Thus, there is a general need for apparatuses supporting the insertion or implantation of medical devices.

Without restricting the scope of the invention, the invention, in the following, specifically will be described with respect to fully or partially insertable medical devices having analytical functions. It shall be noted, however, that, additionally or alternatively, other medical devices are also feasible, such as medical devices usable for medication functions, e.g. for infusion.

Thus, monitoring certain body functions, more particularly monitoring one or more concentrations of at least one analyte such as at least one metabolite in a body fluid, also referred to as a bodily fluid, plays an important role in the prevention and treatment of various diseases. Such analytes can include, by way of example, but not exclusively, glucose, lactate, cholesterol or other types of analytes and metabolites. Without restricting further possible applications, the invention will be described in the following text with reference to glucose monitoring. However, additionally or alternatively, the invention can also be applied to other types of analytes, such as one or more of the analytes mentioned above.

Generally, medical systems for long-term monitoring of an analyte in a body tissue of a user as well as corresponding insertion devices, such as preassembled insertion devices comprising in searches and medical components, are known. These insertion devices may comprise sterile parts for being inserted into the body tissue of the user. Generally, the insertion device may comprise at least one tamper-evident closure to provide indication whether a sterile enclosure of the sterile parts is still intact. In many instances, two different kinds of tamper-evident closures are used. A first kind of tamper-evident closures may comprise sealing elements configured for being destroyed before use, such as when preparing the insertion device for use. A second kind of tamper-evident closure may comprise one or more components of the sterile enclosure itself, which will at least partially be destroyed when removing the sterile enclosure.

As an example, WO 2018/236769 A1 discloses applicators for applying an on-skin assembly to skin of a host and methods of their use and/or manufacture. An applicator includes an insertion assembly configured to insert at least a portion of the on-skin assembly into the skin of the host, a housing configured to house the insertion assembly, the housing comprising an aperture through which the on-skin assembly can pass, an actuation member configured to, upon activation, cause the insertion assembly to insert at least the portion of the on-skin assembly into the skin of the host. The document further discloses a sealing element configured to provide a sterile barrier and a vapor barrier between an internal environment of the housing and an external environment of the housing.

WO 2019/236850 A1 describes a system including a sensor applicator, a sensor control device arranged within the sensor applicator and including an electronics housing and a sensor extending from a bottom of the electronics housing, and a cap coupled to one of the sensor applicator and the sensor control device, wherein the cap is removable prior to deploying the sensor control device from the sensor applicator.

WO 2019/122095 A1 describes a medical system. The medical system comprises: a housing; a preassembled functional module received in the housing, the preassembled functional module comprising an analytical sensor for detecting at least one analyte in a body fluid of a user; an electronics unit electrically connected to the analytical sensor and an insertion component for inserting the analytical sensor into a body tissue of the user; at least one removable protective cap connected to the housing, covering the preassembled functional module.

In the field of medication devices, WO 2014/023771 A1 discloses a drug delivery device for dispensing of a dose of a medicament, comprising a housing to accommodate a cartridge being at least partially filled with a medicament, wherein the housing comprises a dispensing end with an access opening which is closed by a tamper-evident closure.

Sealing elements also are generally known from storage containers. US 5,788,064 A discloses a storage container for test strips with a holder in which the test strips are contained and which is closed by a lid. The lid has an elastic seal on its inner side which, when the storage container is closed presses against a rim of the holder preventing an invasion of moisture into the storage container. Holder and lid are connected to one another by a hinge. When the storage container is closed, the seal is deformed and pushes the lid after release of a locking means such that the storage container is at least partially opened.

Despite the advantages achieved by the methods and devices known in the art, several technical challenges remain. Specifically, a force required for destroying the tamper-evident closure also defines the maximum mechanical stress that may be exerted onto the apparatus in daily use. Thus, in case a force exceeding the maximum payload is accidentally applied to the tamper-evident closure, for example during transport or by accidentally dropping the apparatus, the tamper-evident closure will be accidently destroyed. For apparatuses and insertion devices used in the field of medical systems, in many instances, however, the tamper-evident closure indicates the intactness of the sterile enclosure. Generally, insertion devices are not supposed to be used if the tamper-evident closure is accidently destroyed, so this situation will actually be considered a failure of the insertion device or even of the entire apparatus. Further, specifically in the field of home care, long-term monitoring or self-medication, specific requirements regarding the sealing elements have to be fulfilled, with respect to users other than medically trained personnel. Thus, complex sealing elements as well as sealing elements requiring the application of a manual force beyond the typical capabilities of e.g. elderly people have to be avoided.

### Problem to be solved

It is therefore desirable to provide an apparatus and methods which at least partially address the above-mentioned technical challenges. Specifically, it is desirable to provide an apparatus and methods which provide a safe, stable, user-friendly and reliable tamper-evident closure.

### Summary

This problem is addressed by an apparatus, a method for preparing the apparatus, and a method for inserting at least a part of a medical device of the apparatus, with the features of the independent claims. Advantageous embodiments which might be realized in an isolated fashion or in any arbitrary combinations are listed in the dependent claims, in the list of embodiments and throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the invention, an apparatus is disclosed. Since, in the present case, the apparatus is used for medical purposes, the apparatus may also be referred to as a "medical system". The term "apparatus", as used herein, is a broad term and is to be given its ordinary and customary meaning to a person of ordinary skill in the art and is not to be limited to a special or customized meaning. The term specifically may refer, without limitation, to a device or system comprising a plurality of components. Specifically, at least one component may be configured for performing at least one medical function and/or for being used in at least one medical process, such as one or more of a therapeutic process, a diagnostic process or another medical process. Specifically, as will be outlined in further detail below, the apparatus may comprise at least one component which is configured for being used in qualitatively and/or quantitatively detecting at least one analyte in a body fluid, such as in a body fluid contained in a body tissue of a user. Additionally or alternatively, the apparatus may comprise at least one component configured for delivering of at least one therapeutic fluid into the body tissue of the user. The apparatus may comprise further components providing an insertion mechanism, such as components configured for inserting a medical device into the body tissue of the user. The apparatus specifically may be configured for performing at least one of the following actions: the action of fully or partially inserting the medical device into the body tissue; the action of detecting at least one analyte in a body fluid by using an analyte sensor; the action of delivering of at least one therapeutic fluid into the body tissue. Additionally or alternatively, the medical system may comprise other components, such as components having a protective function and/or another medical function.

The apparatus comprises:
a. a medical device which is at least partially insertable into the body tissue of the user;
b. an insertion device for at least partially inserting the medical device into the body tissue;
c. a housing; and
d. a removable cap connected to the housing, the removable cap being configured for being removed from the housing before insertion of the medical device.

The housing and the removable cap, prior to removal of the removable cap, are connected via at least one flexible connection element. The flexible connection element is reversibly displaceable from a locking position into a releasing position. In the locking position, the removable cap is secured to the housing by the flexible connection element. In the releasing position, the removable cap is released. The flexible connection element, prior to use, is secured in the locking position by at least one frangible securing element.

As outlined above, the apparatus comprises the medical device. The term "medical device" as used herein is a broad term and is to be given its ordinary and customary meaning to a person of ordinary skill in the art and is not to be limited to a special or customized meaning. The term specifically may refer, without limitation, to an arbitrary element, device, system or article being configured for use in the field of medical technology, specifically in at least one field selected from the group consisting of medical analytics, medical diagnostics, medical therapeutics. The medical device may be configured for performing the at least one medical function and/or for being used in at least one medical process, such as one or more of a diagnostic process, a therapeutic process, or another medical process.

As outlined above, the medical device is at least partially insertable into a body tissue of the user. The term "insertable into a body tissue of a user", as used herein, is a broad term and is to be given its ordinary and customary meaning to a person of ordinary skill in the art and is not to be limited to a special or customized meaning. The term specifically may refer, without limitation, to the property of an element or a device being suited for fully or partially being inserted into the body tissue. Specifically, the term may refer to the fact that, at least under conditions of regular use such as during a duration of use of less than a week or less than a month, the insertable element is not subject to substantial deterioration by the body fluid and/or the insertable element does not exert a detrimental effect on to the body tissue, such as by unwantedly releasing unwanted materials, components or substances into the body tissue. For the purpose of being insertable, the insertable element therefore may fully or partially consist of materials which are not subject to deterioration by the body fluid and/or which are not prone to releasing unwanted substances into the body tissue, such as substances causing allergic reactions. Additionally or alternatively, the insertable element may comprise one or more biocompatible surfaces and/or coatings, such as hydrogel coatings or the like. In terms of dimension, the insertable element may be suited for insertion, such as by having dimensions not exceeding 50 mm, more preferably not exceeding 30 mm, in any direction or the like. Other properties supporting insertability may also be given additionally or alternatively.

As outlined above, the medical device is configured for being, at least partially, inserted into the body tissue of the user, such as transcutaneously and/or subcutaneously. Consequently, the medical device may comprise one or more insertable portions or parts. The medical device or at least a part of the medical device may remain in the body tissue of the user for a predetermined period of time, such as for several hours, for one or more days, for up to one week, or for up to two weeks or even more.

As further outlined above, the medical device may be configured for being used in qualitatively and/or quantitatively detecting at least one analyte in the body fluid, such as in the body fluid contained in the body tissue of the user. The medical device may be configured for continuously monitoring and/or detecting the analyte in the body fluid of the user. Alternatively or additionally, the medical device may be configured for delivering the at least one therapeutic fluid into the body tissue of the user.

The term "analyte", as used herein, is a broad term and is to be given its ordinary and customary meaning to a person of ordinary skill in the art and is not to be limited to a special or customized meaning. The term specifically may refer, without limitation, to a chemical and/or biological substance which takes part in the metabolism of a body of the user. Specifically, the analyte may be a metabolite or a combination of two or more metabolites. As an example, the analyte may be selected from the group consisting of: glucose, lactate, triglycerides, cholesterol. Still, other analytes or combinations of two or more analytes may be detected.

The term "user" as used herein is a broad term and is to be given its ordinary and customary meaning to a person of ordinary skill in the art and is not to be limited to a special or customized meaning. The term specifically may refer, without limitation, to a person using the apparatus. The user may be a patient and/or a person intending to insert the medical device in one's own or another's body tissue. Specifically, the user may intend to monitor an analyte, such as a glucose value, in one's own or another's body tissue. Additionally or alternatively, the user may intend to introduce the therapeutic fluid into one's own or another's body tissue. For example, the user may be a patient suffering from a disease, such as diabetes. The body tissue of the user specifically may be or may comprise fatty tissue and/or interstitium. Other types of body tissue, however, are also feasible.

As outlined above, the apparatus comprises the insertion device for at least partially inserting the medical device into the body tissue. The term "insertion device" as used herein is a broad term and is to be given its ordinary and customary meaning to a person of ordinary skill in the art and is not to be limited to a special or customized meaning. The term specifically may refer, without limitation, to a device which is capable of fully or partially implanting or inserting the medical device into the body tissue. For this purpose, the insertion device, specifically and as will be outlined in further detail below, may comprise at least one actuator or driving actuator configured for driving the medical device fully or partially into the body tissue, such as by exerting a force onto the medical device, into a direction of insertion, such as into a direction of a body surface or skin surface of the user. The insertion device specifically may be adapted to transcutaneously or subcutaneously insert the medical device into the body tissue of the user, such as by generating an incision or a puncture in a skin of the user and by transferring the medical device fully or partially into the body tissue. The incision or puncture specifically may be small. Thus, the insertion may be performed by a user without having professional medical expertise and may be performed without substantial physical interaction on the body. As an example, the incision or puncture may have an overall dimension not exceeding 5 mm or even not exceeding 3 mm, and/or a depth of puncture or incision may not exceed 50 mm or even not exceed 30 mm. The insertion device specifically may be a transcutaneous insertion device. As will be outlined in further detail below, the insertion device specifically may be a mechanical insertion device which preferably may be operated by hand, preferably without the need of electrical or electromechanical actuators. However, other embodiments are feasible. The insertion device may enable a retraction of a penetrating member from the body tissue of the user.

The term "at least partially inserting the medical device into the body tissue" as used herein is a broad term and is to be given its ordinary and customary meaning to a person of ordinary skill in the art and is not to be limited to a special or customized meaning. The term specifically may refer, without limitation, to an action of one or more of transcutaneously or subcutaneously applying the medical device to the body tissue of the user. Specifically, the medical device may fully or partially be inserted into the body tissue of the user in such a way that the medical may be able to perform the at least one medical function and/or to be used in the at least one medical process. The action of inserting the medical device may be performed by the user himself, specifically by using the apparatus according to the present invention, such as according to any one of the embodiments disclosed herein and/or disclosed in further detail below. Therein, the term "at least partially" specifically may refer to the fact that the entire medical device may be inserted into the body tissue or that, alternatively, only at least one part or portion of the medical device, also referred to as the insertable portion, may be inserted into the body tissue.

As further outlined above, the apparatus comprises the housing and the removable cap connected to the housing. The term "housing" as used herein is a broad term and is to be given its ordinary and customary meaning to a person of ordinary skill in the art and is not to be limited to a special or customized meaning. The term specifically may refer, without limitation, to an element which is configured for fully or partially enclosing one or more components and for providing protection for these one or more components, such as against mechanical influence and/or humidity. Thus, the housing may comprise one or more interior spaces fully or partially enclosed by at least one wall of the housing.

The housing, specifically, may be or may comprise a rigid housing, such as a rigid housing made of one or more of a plastic material, a metallic material or a cardboard material. Thus, the housing may be a rigid in such a way that one or more components enclosed by the housing are at least widely protected from mechanical influences exerted from the outside onto the apparatus.

As will be outlined in further detail below, the housing may comprise at least one stationary portion which is configured for being disposed on the skin of the user. As an example, the stationary portion may comprise at least one contact surface for contacting the skin of the user, such as an essentially flat surface or a circumferential rim of the stationary part of the housing. Further, in addition to the at least one stationary portion, the housing may comprise at least one actuation portion which is configured for being actuated by the user for insertion of the medical device into the body tissue of the user.

The housing, as will be explained in further detail below, may comprise or may enclose, fully or partially, one or more further components, such as an actuator or the medical device. These one or more further components specifically may be located, as outlined above, in one or more interior spaces fully or partially enclosed by the housing.

The housing basically may have a suited geometrical shape. Specifically, the housing may have a longitudinal extension along at least one axis of extension, such as a longitudinal axis. Thus, as an example, the housing may have a lengthy shape along the axis. The housing specifically may be essentially rotationally symmetric, e.g. by having an axial rotational symmetry about an axis such as a cylinder axis or axis of extension. The housing, as an example, may be designed as a cylinder, a hemisphere or as a dome. Additionally or alternatively, an outer shape of the housing may also be asymmetrical, e.g. may be shaped ergonomically to be held by a human hand. The term "essentially rotationally symmetric" as used herein is a broad term and is to be given its ordinary and customary meaning to a person of ordinary skill in the art and is not to be limited to a special or customized meaning. The term specifically may refer, without limitation, to the fact that a component of the apparatus, for example the housing, may be fully rotationally symmetric or, alternatively, may comprise at least one part being rotationally symmetric, whereas another part of the component may have a form diverging from the rotational symmetry. For example, a lateral surface of the housing may be designed as a cylinder and, thus, may be rotationally symmetric. The housing may comprise further components, such as the actuator, which may specifically not be rotationally symmetric.

The term "removable cap" as used herein is a broad term and is to be given its ordinary and customary meaning to a person of ordinary skill in the art and is not to be limited to a special or customized meaning. The term specifically may refer, without limitation, to an element configured for at least partially covering at least one other device, component or element, thereby providing at least partial protection against mechanical and/or environmental influences, wherein the element is further configured for being removed from the at least one other device, component or element, reversibly or irreversibly. The removable cap specifically may be fully or partially made of at least one rigid material, such as of at least one plastic material and/or at least one metallic material. The removable cap specifically may be made essentially rotationally symmetric, e.g. by having an axial rotational symmetry corresponding to the axial rotational symmetry of the housing. For example, the removable cap may be designed as a cylinder, a hemisphere or as a dome. For being connected to the at least one other device, component or element being covered by the removable cap, the removable cap may comprise one or more connection elements, such as for connecting by at least one of a form-fit or a force-fit connection. As an example, the removable cap may comprise at least one circumferential rim configured for engaging with the at least one device, component or element being covered by the removable cap.

The term "removable" as used herein is a broad term and is to be given its ordinary and customary meaning to a person of ordinary skill in the art and is not to be limited to a special or customized meaning. The term specifically may refer, without limitation, to a characteristic of a device, element or component of the apparatus of being detachable from the apparatus. Specifically, a removable component, such as the removable cap, may be detached by action of the user. For example, the removable cap may be removed by the user from the housing prior to application of the apparatus. The removable cap may be removed from the housing by at least one of: pulling the removable cap off the housing; turning the removable cap off the housing. Specifically, removing the removable cap from the housing may require releasing the removable cap by moving the flexible connection element from the locking position into the releasing position.

As outlined above, the removable cap and the housing, prior to removal of the removable cap, are connected via at least one flexible connection element. The term "flexible connection element" as used herein is a broad term and is to be given its ordinary and customary meaning to a person of ordinary skill in the art and is not to be limited to a special or customized meaning. The term specifically may refer, without limitation, to an element having flexible properties and being configured for connecting at least two elements, specifically at least two elements of the apparatus. Specifically, the flexible connection element may connect the housing and the removable cap of the apparatus via at least one of a form-fit or a force-fit connection. The flexible connection element may form part of at least one of the housing and the removable cap. For example, the flexible connection element may comprise at least one element, such as at least one of a flexible tongue, a hook, a catch or a pin, protruding from the housing and/or from the removable cap towards the corresponding counter element, such as the removable cap or the housing. The flexible connection element may not fixedly connect the housing and the removable cap. The flexible connection element may be movable from the locking position into the releasing position, wherein the connection between the housing and the removable cap may be released if the flexible connection element is in the releasing position. The flexible connection element may prevent or may allow removing of the removable cap. Specifically, the flexible connection element may prevent removing the removable cap when being in the locked position. The flexible connection element may allow removing the removable cap when being in the releasing position.

The term "flexible" as used herein is a broad term and is to be given its ordinary and customary meaning to a person of ordinary skill in the art and is not to be limited to a special or customized meaning. The term specifically may refer, without limitation, to a characteristic of a component or element of the apparatus of being reversibly deformable or displaceable. The flexible element or component may be displaced by action of the user, such as by applying a force to the flexible element. Specifically, the flexible element may have elastic properties and, thus, may deform upon application of a force and may be able to restore to its original state if the external force is no longer applied. The term specifically may refer to elastic properties of the at least one element. Specifically, the term may refer to the property of at least one element of being deformed when a force or a tension is applied to the element, wherein, once the force or tension is removed, the element, at least essentially, resumes its original shape. For example, the flexible element of the apparatus, such as the flexible connection element, may be able to reversibly change one or more of its position and/or its form upon application of a force.

Additionally, the removable cap, as an example, may be connected to the housing by at least one further connection. For example, the removable cap may be connected to the housing via a threaded joint. Thus, both, the housing and the removable cap may have a thread which are compatible with each other. There may be an overlap region in the connected state, in which the removable cap overlaps with the housing or vice versa. As another example, the removable cap may be part of the housing connected by a thin-walled portion with limited strength. Additionally or alternatively, the housing and the removable cap may be further connected via a form-fit or a force-fit connection or via an integral component of the housing as a break-away-part. For example, a rim of the removable cap may be pushed over a rim of the housing or vice a versa. Thus, the removable cap may have a circular, oval or polygonal rim which fits tightly over a rim of the housing having a corresponding shape, or vice versa.

The flexible connection element is reversibly displaceable from a locking position into a releasing position. In the locking position, the removable cap is secured to the housing by the flexible connection element. In the releasing position, the removable cap is released. The term "locking position" as used herein is a broad term and is to be given its ordinary and customary meaning to a person of ordinary skill in the art and is not to be limited to a special or customized meaning. The term specifically may refer, without limitation, to at least one of a position or an orientation of an element, specifically an element of the apparatus, wherein in the locking position or orientation the element may lock or prevent at least one function of the apparatus or any part thereof. Specifically, the locking position may be a position of the flexible connection element in which the removable cap is secured to the housing by the flexible connection element and, thus, in which a removal of the removable cap from the housing is prevented. The locking position may be an initial position of the flexible connection element, such as a position of the flexible connection element prior to use of the apparatus. Generally, the flexible connection element may prevent removing the removable cap from the housing when being in the locking position. The flexible connection element may protrude from the housing or the removable cap and may engage with the corresponding counterpart, wherein the engagement may prevent removing the removable cap. The flexible connection element may, as an example, prevent one or more of pulling or turning off the removable cap from the housing.

Similarly, the term "releasing position" as used herein is a broad term and is to be given its ordinary and customary meaning to a person of ordinary skill in the art and is not to be limited to a special or customized meaning. The term specifically may refer, without limitation, to a position of an element of the apparatus in which the element may allow performing at least one function of the apparatus or any part thereof. Specifically, the releasing position may be a position of the flexible connection element in which the removable cap may be released from the housing, e.g. by the user. The releasing position may be a final position of the flexible element, such as a position after application of a force to the flexible connection element. Further, it may be possible to remove the removable cap when the flexible connection element is in the releasing position. In the releasing position, the further connection of the housing and the removable cap, such as the form-fit or force-fit connection or the threaded joint, may be releasable, such as by turning or by pulling off the removable cap.

As outlined above, the flexible connection element, prior to use, is secured in the locking position by at least one frangible securing element. The term "frangible securing element" as used herein is a broad term and is to be given its ordinary and customary meaning to a person of ordinary skill in the art and is not to be limited to a special or customized meaning. The term specifically may refer, without limitation, to an element configured for performing at least one securing function, wherein the element is configured for being destroyed, such as broken or ruptured, in order to disable the securing function. The term "frangible", thus, refers to the property of the element of being intentionally broken or ruptured, such as by a manual force applicable by the user. Specifically, the frangible securing element may be configured for securing the flexible connection element in the locking position. The frangible securing element may connect the flexible connection element to one of the housing or the removable cap when the flexible connection element is in the locking position. The frangible securing element may be destroyed upon moving the flexible connection element from the locking position into the releasing position, specifically when the flexible connection element is moved from the locking position into the releasing position for the first time. The frangible securing element may indicate whether the flexible connection element has already been moved from the locking position into the releasing position. Specifically, a destroyed frangible securing element may indicate that the flexible connection element has already been moved from the locking position into the releasing position.

The flexible connection element may be movable from the locking position into the releasing position by the user applying a manual force onto the flexible connection element. The frangible securing element may be configured for being destroyed by the manual force, such as by a rupture initiated by a movement of the flexible connection element due to the application of the manual force. The manual force may be applied by the user of the apparatus. The manual force may be applied to the flexible connection element by the user.

A direction of the manual force for moving the flexible connection element from the locking position into the releasing position may be different from a direction of a force required for removing the removable cap from the housing. Consequently, since the directions of forces may be different, the unlocking of the flexible connection element and the removing of the removable cap may require different handling actions or different handling steps, which may be performed simultaneously, subsequently our in a timely overlapping fashion, which, however, may be separate. Specifically, an attempt of the user to remove the removable cap from the housing may be unsuccessful unless the flexible connection element, previously, has been moved from the locking position into the releasing position, since the movements may be separate. Thereby, and unintended removal of the cap may be prevented. The term "direction", as used herein in the context of a force, is a broad term and is to be given its ordinary and customary meaning to a person of ordinary skill in the art and is not to be limited to a special or customized meaning. The term specifically may refer, without limitation, to an orientation, a mean orientation or a temporary orientation of a vector describing the force. Therein, the term "force" may generally imply a force as a vector indicating size and direction of the force applied, but may also imply other means of describing forces or moments being exerted onto at least one element, such as a torque.

The apparatus, specifically, the housing and the removable cap, may have a cylindrical shape and, thus, may extend along a longitudinal axis. The direction of the manual force may be a radial direction with respect to the longitudinal axis of the apparatus. The direction of force required for removing the cap may comprise a direction perpendicular to the radial direction, such as a longitudinal direction essentially parallel to the longitudinal axis or a rotational direction about the longitudinal axis, such as by applying a torque having a torque vector essentially parallel to the longitudinal axis. The term "essentially", as used therein, may also imply slight deviations from a precise parallel orientation, such as implying tolerances of up to 10°, preferably of no more than 5°.

The flexible connection element, e.g. due to its elastic properties, may be configured for returning from the releasing position back into the locking position by an elastic restoring force when the manual force is released. The flexible connection element may comprise at least one force application surface accessible to the user. The manual force may be applicable via the force application surface. The force application surface may comprise an indentation in an outer surface of the apparatus, specifically an indentation in an outer surface of the flexible connection element. The indentation may be configured for receiving a finger tip of the user. The indentation may provide an indication to the user where to apply the manual force to the flexible connection element.

The flexible connection element may be at least partially integrated into one of the removable cap and the housing. The term "at least partially integrated" as used herein is a broad term and is to be given its ordinary and customary meaning to a person of ordinary skill in the art and is not to be limited to a special or customized meaning. The term specifically may refer, without limitation, to the fact that an element of the apparatus may be fully integrated into another element or, alternatively, that only a part of the element may be integrated into the other element. For example, the flexible connection element may be fully integrated into one of the housing and the removable cap. Alternatively, a part of the flexible connection element may not be integrated into one of the housing and the removable cap, whereas the other part may be comprised by the one of the housing and the removable cap.

The flexible connection element may further be configured for remaining with the removable cap or the housing, respectively, after removing the removable cap from the housing. Thus, as an example, the flexible connection element may still be connected to the housing or to the removable cap, respectively, after removal of the removable cap from the housing. As an example, the flexible connection element may still be an integral part of the housing or to the removable cap, respectively, after removal of the removable cap from the housing.

The flexible connection element may be flexibly displaceable by at least one of manually pressing, twisting or pulling the flexible connection element. The flexible connection element may comprise at least one of a flexible tongue, a hook, a catch or a pin. Specifically, the flexible connection element may comprise the at least one flexible tongue. The flexible tongue may comprise at least one free end and at least one fixed end. The fixed end may be connected to one of the housing and the removable cap. The free end may be configured for engaging with at least one of the housing and the removable cap. The frangible securing element may secure the flexible tongue in the locking position. The flexible tongue may be at least partially received within a slot of one of the housing and the removable cap. The frangible securing element may secure the flexible tongue to at least one edge of the slot.

The flexible connection element, as indicated above, may be connected to, specifically integrated into, one of the housing and the removable cap. The other one of the housing and the removable cap may comprise at least one depression configured for engaging with the flexible connection element in the locking position. For example, the flexible connection element may comprise the at least one flexible tongue having the at least one free end. The free end of the flexible tongue may be configured for engaging with the depression of the housing or the removable cap in the locking position.

Specifically, the flexible connection element may be part of the removable cap and may be configured to engage with the housing in the locking position. The flexible connection element may engage with the depression of the housing. The flexible connection element may be flexibly displaceable into the releasing position by disengaging with the housing.

The frangible securing element may comprise at least one frangible ligament securing at least a part of the flexible connection element in the locking position. The frangible securing element may secure the flexible connection element in the locking position to the removable cap. As an example, the flexible connection element, the frangible securing element and one of the housing and the removable cap may be made as an integral element, before displacing the flexible connection element from the locking position into the releasing position and, thereby, breaking the frangible securing element. As an example, as outlined above, the flexible connection element may comprise at least one tongue connected to one of the housing or the removable cap. The frangible securing element may comprise a ligament connecting the tongue to the housing or removable cap, respectively, before the ligament is broken by exerting the manual force. The ligament may hold the tongue in a specific position, i.e. in the locking position, before the ligament is broken. For example, the tongue, the ligament and the housing or removable cap, respectively, may be molded as a single piece, e.g. injection molded.

The flexible connection element may be movable from the locking position into the releasing position in a releasing direction. The releasing direction may be a radial direction with respect to the longitudinal axis of the apparatus, i.e. a direction essentially perpendicular to a longitudinal axis. Again, the term "essentially" may imply tolerances, such as angular tolerances of no more than 10°, specifically of no more than 5°. During removing from the housing, the removable cap may be movable into at least one removing direction. The releasing direction may be different from the removing direction. For example, the removing direction may comprise a direction perpendicular to the releasing direction, such as a direction essentially parallel to the longitudinal axis of the apparatus or a rotational direction about the longitudinal axis, as outlined above.

The apparatus may be configured such that bringing the flexible connection element from the locking position into the releasing position and removing of the removable cap from the housing may require separate mechanical actions by the user. For example, the removable cap may be configured such that removing the removable cap from the housing may require a twisting motion of the removable cap, specifically a twisting motion of the removable cap about the longitudinal axis of the apparatus. Thus, removing the removable cap may require a mechanical action of twisting the removable cap. As outlined above, bringing the flexible connection element from the locking position into the releasing position may require application of the manual force. Thus, bringing the flexible connection element from the locking position into the releasing position may require a mechanical action of applying the manual force to the flexible connection element, such as by manually pressing, twisting or pulling the flexible connection element.

The housing and the removable cap, prior to removal of the removable cap, may be locked to each other via the flexible connection element by at least one of a force-fit and a form-fit connection. The removable cap may be connected to the housing by a twistable connection, specifically by at least one screw coupling, such as a threaded joint. At least one of the removable cap and the housing may comprise at least one screw thread. Specifically, both of the housing and the removable cap may comprise screw threads corresponding to each other, such that the screw threads may form the twistable connection between the housing and the removable cap.

As outlined above, various options are feasible for the medical device, which may also be combined. Thus, the medical device may comprise at least one of: a device for delivery of at least one therapeutic fluid into the body tissue of the user, specifically at least one infusion set comprising at least one infusion cannula; at least one analyte sensor for detecting at least one analyte; specifically at least one analyte sensor for detecting at least one bodily analyte in the at least one bodily fluid, more specifically at least one electrochemical analyte sensor comprising at least one sensor electrode for detecting the at least one analyte.

The term "infusion set" as used herein is a broad term and is to be given its ordinary and customary meaning to a person of ordinary skill in the art and is not to be limited to a special or customized meaning. The term specifically may refer, without limitation, to a device and/or a combination of devices configured for delivering at least one fluid, such as at least one therapeutic fluid, into the body tissue of the user. For example, the infusion set may comprise at least one infusion cannula, such as a soft infusion cannula, e.g. in infusion cannula made of an elastomeric material such as silicone, and/or a hard infusion cannula, such as a hard infusion cannula made of a rigid material such as a metal and/or a rigid plastic material. The infusion set may further comprise at least one tubing connected to the infusion cannula. Further, the infusion set may comprise one or more other components, such as one or more connectors for connecting the tubing to the cannula and/or one or more plasters or other adhesive element for adhering the infusion set to a body surface or skin of the user. The infusion set may further comprise one or more connectors for connecting the infusion set to at least one dispensing device, such as a pump or the like, configured for dispensing the therapeutic fluid through the infusion cannula into the body tissue of the user. The infusion cannula of the infusion set may be or may comprise at least one hollow needle or hollow tube configured for delivering and/or infusing the therapeutic fluid into the body tissue of the user.

The term "analyte sensor" as used herein is a broad term and is to be given its ordinary and customary meaning to a person of ordinary skill in the art and is not to be limited to a special or customized meaning. The term specifically may refer, without limitation, to a sensor which is capable of qualitatively or quantitatively detecting the presence and/or the concentration of at least one analyte in the body tissue and/or in a body fluid contained within the body tissue. Specifically, the analyte sensor may be an electrochemical analyte sensor, having at least two sensor electrodes, such as at least one working electrode and at least one further electrode such as at least one counter electrode and/or at least one reference electrode. The working electrode may comprise a working electrode pad and, optionally, at least one test chemical disposed thereon. The at least one further electrode, such as the at least one counter electrode and/or the at least one reference electrode, may comprise a conductive electrode pad. Additionally and optionally, one or more redox materials may be disposed thereon. Potential embodiments of analyte sensors, specifically for insertion into a body tissue and more specifically for continuous monitoring or long-term monitoring, are generally known to the skilled person. As an example, reference may be made to the basic basic principles of test elements and reagents described e.g. in J. Hones et al.: Diabetes Technology and Therapeutics, Vol. 10, Supplement 1, 2008, pp. 10-26. Other embodiments of the analyte sensor may be possible too. For example, the analyte sensor may comprise a flexible light guide with glucose sensitive coating at its end and/or a tube like carrier with functional elements at inner or outer walls. Generally, as an example, the analyte sensor may comprise an electrochemical analyte sensor. Thus, as an example, the analyte sensor may be a strip-shaped analyte sensor having a flexible substrate and the at least two electrodes disposed thereon. As an example, the analyte sensor may have a length of 5 mm to 50 mm, specifically a length of 7 mm to 30 mm. The analyte sensor may further provide a biocompatible cover, such as a biocompatible membrane which fully or partially covers the analyte sensor and which prevents the test chemical from migrating into the body tissue and which allows for a diffusion of the body fluid and/or the analyte to the electrodes. The analyte sensor may further provide one or more leads for electrically contacting the electrodes. The leads may, during insertion or at a later point in time, be connected to an electronics unit, such as one or more measurement devices adapted for measuring electrical currents and/or electrical voltages, such as to one or more potentiostats.

The medical device may be at least partially accommodated within the housing. The insertion device may comprise at least one actuator for driving the medical device into the body tissue, specifically at least one actuable displacement mechanism which upon actuation is capable of driving and/or protracting the medical device towards the body tissue of the user, with the driving and/or protraction resulting in at least partially inserting the medical device into the body tissue.

The term "actuator" as used herein is a broad term and is to be given its ordinary and customary meaning to a person of ordinary skill in the art and is not to be limited to a special or customized meaning. The term specifically may refer, without limitation, to an arbitrary device which is configured for directly or indirectly inserting at least one insertable element into the body tissue of the user, e.g. by transferring a driving force directly or indirectly onto the insertable element. Specifically, the driving force may be transferred to a penetrating member, such as an insertion cannula or an insertion needle. The actuator may comprise at least one mechanical actuator for transferring one or both of a manually applied driving force or a force provided by a mechanical element such as a spring onto the penetrating member. The actuator may comprise at least one mechanical device which is configured for driving forward the penetrating member into the body tissue. The actuator, as an example, may comprise at least one slider connected to the penetrating member or a holder for the penetrating member and may be configured for performing a forward linear motion in an insertion direction and, optionally, a backward linear motion in an opposite direction. As an example, the slider may be driven by at least one spring element, which may be pre-tensioned or biased in the forward direction or in the backward direction. Additionally or alternatively, the slider may be connected to at least one actuation button which may be pushed by the user, thereby driving the slider in the forward direction.

The actuator may be a manual actuator which is manually actuatable by the user. The actuator may comprise at least one of the following: an insertion spring for driving the medical device into the body tissue; a return spring for retracting the penetrating member from the body tissue after insertion, specifically by leaving the inserted medical device in place. The actuator, as an example, may comprise one or more actuation elements which may directly or indirectly be actuated by the user, such as one or more buttons to be pushed by the user and/or one or more levers to be tilted or pivoted by the user, thereby allowing the user to exert an actuation force via the actuator, including the option of exerting an actuation torque.

The insertion device, specifically the actuator, may comprise at least one part of the housing. The housing may comprise at least one stationary portion configured for resting on the skin of the user and at least one actuation portion configured for being manually actuated by the user for actuating the actuator. As outlined above, the actuation portion, as an example, may comprise at least one of a button configured for being pushed by the user and/or at least one lever configured for being tilted or pivoted by the user. Other options are feasible. The stationary portion and the actuation portion may be movable with respect to each other. The stationary portion and the actuation portion each may comprise cylindrical parts which are concentrically disposed with respect to the longitudinal axis of the apparatus. Thus, as an example, the actuation portion may be configured for sliding essentially parallel to the longitudinal axis within the stationary portion, or vice versa. This embodiment provides for a stable actuation, since the stationary portion stabilizes the movement of the actuation portion. Other embodiments, however, are also generally feasible.

The insertion device, specifically the actuator, may comprise the at least one return spring for retracting the penetrating member from the body tissue after insertion. The penetrating member may be part of the apparatus, specifically of the insertion device. The penetrating member may be configured for inserting the medical device into the body tissue. When retracting the penetrating member, the penetrating member may further be configured for leaving the medical device in the body tissue of the user.

The medical device may be comprised by at least one preassembled functional module of the apparatus, specifically by at least one penetrating cartridge. The at least one preassembled functional module may comprise, besides the at least one medical device, specifically besides the at least one analyte sensor, at least one of the following components: the at least one penetrating member for inserting the medical device into the body tissue, more specifically at least one insertion needle; at least one infusion cannula for delivery of at least one therapeutic fluid into the body tissue of the user; at least one electronics unit electrically connected to the medical device, specifically at least one electronics unit for sensing the at least one analyte in conjunction with the at least one analyte sensor.

The term "preassembled functional module" as used herein is a broad term and is to be given its ordinary and customary meaning to a person of ordinary skill in the art and is not to be limited to a special or customized meaning. The term specifically may refer, without limitation, to a module, such as a unitary module made of one or more components, specifically a plurality of interconnected components, which are configured for interacting for performing at least one function and/or for being used for performing at least one function, specifically at least one medical function. In the present case, the functional module specifically may be a medical functional module configured for performing at least one medical function, such as for qualitatively and/or quantitatively detecting the at least one analyte in a body fluid and/or for delivering the at least one therapeutic fluid into the body tissue of the user. The preassembled functional module may be received in the housing of the apparatus.

The term "preassembled" as used herein is a broad term and is to be given its ordinary and customary meaning to a person of ordinary skill in the art and is not to be limited to a special or customized meaning. The term specifically may refer, without limitation, to a state or property of a device having already completed a full or partial assembly process. Thus, the components of the preassembled functional module may already be assembled, such as by being mechanically and/or electrically interconnected, thereby being ready for use for the at least one function, such as the at least one medical function. The preassembling specifically may take place in a factory, thereby rendering the preassembled functional module a factory-assembled functional module. The preassembling specifically may take place under specific conditions, such as under specific germ-free or sterile conditions, whereas the remaining assembly of the apparatus may take place under different conditions, such as conditions not having significant requirements regarding sterility. Thus, the effort of assembly may significantly be reduced by using the preassembled functional module. The apparatus may be configured for receiving the preassembled function module, such that the preassembled functional module may be fully covered by the combination of the housing and the removable cap, such that the user may not see or manipulate the preassembled functional module without opening the apparatus, e.g. without removing the removable cap.

The term "penetrating member" as used herein is a broad term and is to be given its ordinary and customary meaning to a person of ordinary skill in the art and is not to be limited to a special or customized meaning. The term specifically may refer, without limitation, to an element which may be at least partially insertable into the body tissue, particularly in order to deliver or to transfer a further element. The penetrating member may be configured to receive another element at least partially, such as to receive the medical device, for example the analyte sensor or the infusion cannula. Thus, the penetrating member may be configured to support an insertion of the medical device, such as of the analyte sensor or of the infusion cannula as described above or as will further be described below. The penetrating member may be or may comprise an insertion needle, such as a compact needle, and/or an insertion cannula, such as a hollow cannula. The penetrating member may comprise a tip or a sharp and may assist in inserting the medical device into the body tissue of the user, specifically for inserting the medical device subcutaneously or transcutaneously. The medical device may be configured for remaining in the body tissue of the user. The penetrating member may be configured for being retracted after the medical device was inserted into the body tissue of the user leaving the inserted medical device in the body tissue of the user. Specifically, the insertion device may be configured for retracting the penetrating member after the medical device was inserted into the body tissue of the user.

As further used herein, the term "insertion cannula" is a broad term and is to be given its ordinary and customary meaning to a person of ordinary skill in the art. An insertion cannula may refer to a hollow needle which may be at least partially slotted. The medical device may then typically be received within the insertion cannula, meaning within the hollow part, such as within a lumen of the insertion cannula.

As further used herein, the term "insertion needle" is a broad term and is to be given its ordinary and customary meaning to a person of ordinary skill in the art. An insertion needle may refer to a compact needle, specifically without a slot and without any hollow parts. The medical device may then typically be received on an outer surface of the insertion needle.

The term "electronics unit" as used herein is a broad term and is to be given its ordinary and customary meaning to a person of ordinary skill in the art and is not to be limited to a special or customized meaning. The term specifically may refer, without limitation, to a unit, such as a unit which may be handled as a single piece, which is configured for performing at least one electronic function. Specifically, the electronics unit may have at least one interface for being connected to the medical device, specifically to the analyte sensor, wherein the electronics unit may provide at least one electronic function interacting with the medical device, such as at least one measurement function. The electronics unit specifically may be configured for measuring at least one voltage and/or for measuring at least one current, thereby interacting with the medical device, specifically with the analyte sensor. The electronics unit may further comprise at least one integrated circuit, such as a processor and/or a microcontroller. The term "processor", as well as the term "microcontroller" as generally used herein are broad terms and are to be given their ordinary and customary meaning to a person of ordinary skill in the art and is not to be limited to a special or customized meaning. The terms specifically may refer, without limitation, to a device which is configured for performing calculations or logic operations. In particular, the processor may be configured for processing an electronic signal, such as a current or a voltage, specifically an electronic signal from the medical device. Specifically, the processor and/or microcontroller may be or may comprise a central processing unit (CPU). Additionally or alternatively, the processor and/or microcontroller may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like. The processor and/or microcontroller specifically may be configured, such as by software programming, for performing one or more evaluation operations. Thus, the processor and/or microcontroller may be configured for processing and/or evaluating the electronic signal from the medical device and, for example, outputting a signal indicating the analyte concentration measured by the analyte sensor. The electronics unit further may comprise at least one measuring device for measuring at least one of a voltage and a current, such as a potentiostat. The processor and/or the microcontroller specifically may be configured for controlling one or more electronic functions of the electronics unit.

The insertion device may be configured for moving the preassembled functional module towards the skin of the user, specifically resulting in the at least partial insertion of the medical device into the body tissue. The preassembled function module may further comprise at least one adhesive means for being attached to a skin site of the user, such as a plaster or the like.

The preassembled functional module may comprise at least one protective cap, specifically at least one sterility cap. The term "protective cap" as used herein is a broad term and is to be given its ordinary and customary meaning to a person of ordinary skill in the art and is not to be limited to a special or customized meaning. The term specifically may refer, without limitation, to an element such as a cover which is configured for maintaining a sterile atmosphere in an interior space fully or partially surrounded by the element. Specifically, the protective cap may provide a sealed interior space, wherein the sealed interior space may at least partially surround the medical device and/or the penetrating member. The protective cap may comprise an outer contour, such as one or more notches, grooves, protrusions or the like, forming an interlocking with the removable cap, specifically with an inner contour of the removable cap.

The apparatus may be configured such that removing the removable cap from the housing also removes the protective cap from the preassembled functional module, specifically by twist-off. The removable cap may comprise the at least one inner contour configured for engaging with the outer contour of the protective cap. The removable cap may be configured for being removed from the housing by a rotational movement, wherein the removable cap may be configured for transferring a torque and/or a force onto the protective cap during removal. The protective cap may be removed from the preassembled functional module by the torque transferred to the protective cap.

The apparatus may further comprise at least one removable protective cap, specifically at least one removable protective cap connected to the preassembled functional module. The cylindrical removable cap may be configured for removing the removable protective cap when being removed from the housing. The cylindrical removable cap may comprise at least one engaging contour for engaging with a corresponding contour of the removable protective cap.

The housing may comprise at least one sheath with a distal side facing the skin and a proximal side facing away from the skin of the user. The insertion device may be configured for placement onto a skin surface of the user, specifically by a circumferential rim of the housing. The medical device may comprise at least one portion which is at least one of transcutaneously or subcutaneously insertable into the body tissue.

The housing may be at least partially cylindrical with respect to the longitudinal axis of the apparatus. The removable cap may be at least partially cylindrical. The removable cap may be located concentrically with respect to the longitudinal axis of the apparatus. The removable cap may cover at least a distal side of the housing prior to placement of the apparatus onto the skin of the user.

In a further aspect of the present invention, a method for preparing the apparatus for insertion of at least a part of the medical device into a body tissue of a user is disclosed. The apparatus may be embodied according to any one of the embodiments disclosed above and/or according to any one of the embodiments disclosed in further detail below. The method comprises displacing the flexible connection element from the locking position into the releasing position, thereby at least partially destroying the frangible securing element. The method further comprises removing the removable cap from the housing. As outlined above, these method steps may be performed sequentially or in a timely overlapping fashion. Specifically, the displacing of the flexible connection element from the locking position into the releasing position may be performed or started at least partially before removing the removable cap from the housing.

In a further aspect of the present invention, a method for at least partially inserting at least a part of the medical device of the apparatus into a body tissue of a user is disclosed. Again, the apparatus may be embodied according to any one of the embodiments disclosed above and/or according to any one of the embodiments disclosed in further detail below. The method comprises preparing the apparatus for insertion by using the method for preparing the apparatus according to the present invention, such as according to any one of the embodiments disclosed above and/or according to any one of the embodiments disclosed in further detail below. The method further comprises placing a distal end of the housing onto the skin of the user and actuating the insertion device. The method may further comprise actuating the insertion device, thereby having the insertion device insert the medical device into the body tissue.

The methods and device according to the present invention may provide a large number of advantages over known methods and devices. Specifically, the apparatus comprising the flexible connection element and the frangible securing element may provide a tamper-evident closure, wherein the manual force required for moving the flexible connection element may be different from the force required to remove the removable cap of the apparatus. The flexible connection element may prevent a movement of the removable cap in the locking position, specifically the twisting of the removable cap. When the flexible connection element is moved into the releasing position by applying the manual force, removing of the removable cap, specifically twisting the removable cap, may be possible. The movement of the flexible connection element from the locking position into the releasing position may be a direct movement or an indirect movement, supported by an additional securing element. Further, by releasing the movement of the removable cap, a rotational movement and/or a translational movement of the removable cap or any combination thereof may be possible.

The apparatus according to the present invention may provide means for separating the manual force required to move the flexible connection element from the locking position into the releasing position from the force required to remove the removable cap from the housing. The force required to remove the cap from the housing may exceed the manual force required to destroy the frangible securing element. Further, irrespective of a suitable production design of the apparatus, the flexible connection element and the frangible securing element may be designed such that the user may not be able to accidentally actuate the flexible connection element.

Further, the apparatus may allow a secure positioning of the removable cap on the housing, specifically by using the removable cap screwed on the housing. The flexible connection element, specifically in conjunction with the frangible securing element, may provide means for detection of tampering of the apparatus.

Summarizing and without excluding further possible embodiments, the following embodiments may be envisaged:
Embodiment 1: An apparatus, specifically a medical system, comprising:
   a. a medical device which is at least partially insertable into a body tissue of a user;
   b. an insertion device for at least partially inserting the medical device into the body tissue;
   c. a housing; and
   d. a removable cap connected to the housing, the removable cap being configured for being removed from the housing before insertion of the medical device,
   wherein the housing and the removable cap, prior to removal of the removable cap, are connected via at least one flexible connection element, wherein the flexible connection element is reversibly displaceable from a locking position in which the removable cap is secured to the housing by the flexible connection element into a releasing position in which the removable cap is released, wherein the flexible connection element, prior to use, is secured in the locking position by at least one frangible securing element.
Embodiment 2: The apparatus according to any one of the preceding embodiments, wherein the flexible connection element is movable from the locking position into the releasing position by applying a manual force, wherein the frangible securing element is configured for being destroyed by the manual force.
Embodiment 3: The apparatus according to the preceding embodiment, wherein a direction of the manual force is different from a direction of a force required for removing the removable cap from the housing.
Embodiment 4: The apparatus according to any one of the two preceding embodiments, wherein the flexible connection element, due to its elastic properties, is configured for returning from the releasing position back into the locking position by an elastic restoring force when the manual force is released.
Embodiment 5: The apparatus according to any one of the three preceding embodiments, wherein the flexible connection element comprises at least one force application surface accessible to the user, wherein the manual force is applicable via the force application surface.
Embodiment 6: The apparatus according to the preceding embodiment, wherein the force application surface comprises an indentation in an outer surface of the apparatus, the indentation being configured for receiving a finger tip of the user.
Embodiment 7: The apparatus according to any one of the preceding embodiments, wherein the flexible connection element is at least partially integrated into one of the removable cap and the housing, wherein the flexible connection element is further configured for remaining with the removable cap or the housing, respectively, after removing the removable cap from the housing.
Embodiment 8: The apparatus according to any one of the preceding embodiments, wherein the flexible connection element is flexibly displaceable by at least one of manually pressing, twisting or pulling the flexible connection element.
Embodiment 9: The apparatus according to any one of the preceding embodiments, wherein the flexible connection element comprises at least one of a flexible tongue, a hook, a catch or a pin.
Embodiment 10: The apparatus according to any one of the preceding embodiments, wherein the flexible connection element comprises at least one flexible tongue.
Embodiment 11: The apparatus according to the preceding embodiment, wherein the flexible tongue comprises at least one free end and at least one fixed end, the fixed end being connected to one of the housing and the removable cap.
Embodiment 12: The apparatus according to any one of the two preceding embodiments, wherein the frangible securing element secures the flexible tongue in the locking position.
Embodiment 13: The apparatus according to any one of the three preceding embodiments, wherein the flexible tongue is at least partially received within a slot of one of the housing and the removable cap, wherein the frangible securing element secures the flexible tongue to at least one edge of the slot.
Embodiment 14: The apparatus according to any one of the preceding embodiments, wherein the flexible connection element is connected to, specifically integrated into, one of the housing and the removable cap.
Embodiment 15: The apparatus according to the preceding embodiment, wherein the other one of the housing and the removable cap comprises at least one depression configured for engaging with the flexible connection element in the locking position.
Embodiment 16: The apparatus according to any one of the preceding embodiments, wherein the flexible connection element is part of the removable cap and is configured to engage with the housing in the locking position and wherein the flexible connection element is flexibly displaceable into the releasing position by disengaging with the housing.
Embodiment 17: The apparatus according to any one of the preceding embodiments, wherein the frangible securing element comprises at least one frangible ligament securing at least a part of the flexible connection element in the locking position.
Embodiment 18: The apparatus according to any one of the preceding embodiments, wherein the frangible securing element secures the flexible connection element in the locking position to the removable cap.
Embodiment 19: The apparatus according to any one of the preceding embodiments, wherein the flexible connection element is movable from the locking position into the releasing position in a releasing direction.
Embodiment 20: The apparatus according to the preceding embodiment, wherein the releasing direction is a radial direction with respect to a longitudinal axis of the apparatus.
Embodiment 21: The apparatus according to any one of the two preceding embodiments, wherein the removable cap is, during removing from the housing, movable into at least one removing direction, wherein the releasing direction is different from the removing direction.
Embodiment 22: The apparatus according to any one of the preceding embodiments, wherein the apparatus is configured such that bringing the flexible connection element from the locking position into the releasing position and removing of the removable cap from the housing requires separate mechanical actions by the user.
Embodiment 23: The apparatus according to any one of the preceding embodiments, wherein the removable cap is configured such that removing the removable cap from the housing requires a twisting motion of the removable cap, specifically a twisting motion of the removable cap about a longitudinal axis of the apparatus.
Embodiment 24: The apparatus according to any one of the preceding embodiments, wherein the housing and the removable cap, prior to removal of the removable cap, are locked to each other via the flexible connection element by at least one of a force-fit and a form-fit connection.
Embodiment 25: The apparatus according to any one of the preceding embodiments, wherein the removable cap is connected to the housing by a twistable connection, specifically by at least one screw coupling.
Embodiment 26: The apparatus according to any one of the preceding embodiments, wherein at least one of the removable cap and the housing comprises at least one screw thread.
Embodiment 27: The apparatus according to any one of the preceding embodiments, wherein the medical device comprises at least one of a device for delivery of at least one therapeutic fluid into a body tissue of a user, specifically at least one infusion set comprising at least one infusion cannula; at least one analyte sensor for detecting at least one analyte; specifically at least one analyte sensor for detecting at least one bodily analyte in the at least one bodily fluid, more specifically at least one electrochemical analyte sensor comprising at least one sensor electrode for detecting the at least one analyte.
Embodiment 28: The apparatus according to any one of the preceding embodiments, wherein the medical device is at least partially accommodated within the housing.
Embodiment 29: The apparatus according to any one of the preceding embodiments, wherein the insertion device comprises at least one actuator for driving the medical device into the body tissue, specifically at least one actuable displacement mechanism which upon actuation is capable of driving and/or protracting the medical device towards the body tissue of the user, with the driving and/or protraction resulting in at least partially inserting the medical device into the body tissue.
Embodiment 30: The apparatus according to the preceding embodiment, wherein the actuator is a manual actuator which is manually actuatable by the user.
Embodiment 31: The apparatus according to any one of the two preceding embodiments, wherein the actuator comprises at least one of the following: an insertion spring for driving the medical device into the body tissue; a return spring for retracting a penetrating member from the body tissue after insertion, specifically by leaving the inserted medical device in place.
Embodiment 32: The apparatus according to any one of the preceding embodiments, wherein the insertion device, specifically the actuator, comprises at least one part of the housing.
Embodiment 33: The apparatus according to the preceding embodiment, wherein the housing comprises at least one stationary portion configured for resting on the skin of the user and at least one actuation portion configured for being manually actuated by the user for actuating the actuator.
Embodiment 34: The apparatus according to the preceding embodiment, wherein the stationary portion and the actuation portion are movable with respect to each other.
Embodiment 35: The apparatus according to any one of the two preceding embodiments, wherein the stationary portion and the actuation portion each comprise cylindrical parts which are concentrically disposed with respect to a longitudinal axis of the apparatus.
Embodiment 36: The apparatus according to any one of the preceding embodiments, wherein the insertion device comprises at least one return spring for retracting a penetrating member from the body tissue after insertion.
Embodiment 37: The apparatus according to the preceding embodiments, wherein the penetrating member is part of the apparatus, specifically of the insertion device.
Embodiment 38: The apparatus according to any one of the two preceding embodiments, wherein the penetrating member is configured for inserting the medical device into the body tissue, wherein, when retracting the penetrating member, the penetrating member is further configured for leaving the medical device in the body tissue.
Embodiment 39: The apparatus according to any one of the preceding embodiments, wherein the medical device is comprised by at least one preassembled functional module of the apparatus, specifically by at least one penetrating cartridge, the at least one preassembled functional module comprising, besides the at least one medical device, specifically besides the at least one analyte sensor, at least one of the following components: at least one penetrating member for inserting the medical device into the body tissue, more specifically at least one insertion needle; at least one infusion cannula for delivery of at least one therapeutic fluid into a body tissue of a user; at least one electronics unit electrically connected to the medical device, specifically at least one electronics unit for sensing the at least one analyte in conjunction with the at least one analyte sensor.
Embodiment 40: The apparatus according to the preceding embodiment, wherein the insertion device is configured for moving the preassembled functional module towards the skin of the user, specifically resulting in the at least partial insertion of the medical device into the body tissue.
Embodiment 41: The apparatus according to any one of the two preceding embodiments, wherein the preassembled functional module comprises at least one protective cap, specifically at least one sterility cap, wherein the apparatus is configured such that removing the removable cap removes the protective cap from the preassembled functional module, specifically by twist-off.
Embodiment 42: The apparatus according to the preceding embodiment, wherein the removable cap comprises at least one inner contour configured for engaging with an outer contour of the protective cap.
Embodiment 43: The apparatus according to any one of the two preceding embodiments, wherein the removable cap is configured for being removed from the housing by a rotational movement, wherein the removable cap is configured for transferring a torque onto the protective cap during removal.
Embodiment 44: The apparatus according to any one of the preceding embodiments, further comprising at least one removable protective cap, specifically at least one removable protective cap connected to the preassembled functional module, wherein the cylindrical removable cap is configured for removing the removable protective cap when being removed from the housing.
Embodiment 45: The apparatus according to the preceding embodiment, wherein the cylindrical removable cap comprises at least one engaging contour for engaging with a corresponding contour of the removable protective cap.
Embodiment 46: The apparatus according to any one of the preceding embodiments, wherein the housing comprises at least one sheath with a distal side facing the skin and a proximal side facing away from the skin of the user.
Embodiment 47: The apparatus according to any one of the preceding embodiments, wherein the insertion device is configured for placement onto a skin surface of the user, specifically by a circumferential rim of the housing.
Embodiment 48: The apparatus according to any one of the preceding embodiments, wherein the medical device comprises at least one portion which is at least one of transcutaneously or subcutaneously insertable into the body tissue.
Embodiment 49: The apparatus according to any one of the preceding embodiments, wherein the housing is at least partially cylindrical with respect to a longitudinal axis of the apparatus.
Embodiment 50: The apparatus according to any one of the preceding embodiments, wherein the removable cap is at least partially cylindrical.
Embodiment 51: The apparatus according to any one of the preceding embodiments, wherein the removable cap is located concentrically with respect to a longitudinal axis of the apparatus.
Embodiment 52: The apparatus according to any one of the preceding embodiments, wherein the removable cap covers at least a distal side of the housing prior to placement of the apparatus onto the skin of the user.
Embodiment 53: A method for preparing the apparatus according to any one of the preceding embodiments for insertion of at least a part of the medical device into a body tissue of a user, the method comprising displacing the flexible connection element from the locking position into the releasing position, thereby at least partially destroying the frangible securing element, the method further comprising removing the removable cap from the housing.
Embodiment 54: A method for at least partially inserting at least a part of the medical device of the apparatus according to any one of the preceding embodiments referring to an apparatus into a body tissue of a user, the method comprising preparing the apparatus for insertion by using the method according to the preceding embodiment, the method further comprising placing a distal end of the housing onto the skin of the user and actuating the insertion device.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a schematic embodiment of an apparatus in a cross-sectional view;
- Figure 2: shows the embodiment of the apparatus with a removable cap connected to the housing in a perspective view;
- Figure 3: shows the embodiment of the apparatus without the removable cap in a perspective view;
- Figure 4: shows the removable cap of the embodiment of the apparatus in a perspective view;
- Figure 5: shows a first embodiment of a flexible connection element in a cross-sectional view;
- Figure 6: shows a second embodiment of the flexible connection element in a cross-sectional view;
- Figure 7: shows a third embodiment of the flexible connection element in a cross-sectional view;
- Figure 8: shows a flow chart of an embodiment of a method for preparing the apparatus for insertion of at least a part of a medical device into a body tissue of a user; and
- Figure 9: shows a flow chart of an embodiment of a method for at least partially inserting at least a part of the medical device of the apparatus into the body tissue of the user.

### Detailed description of the embodiments

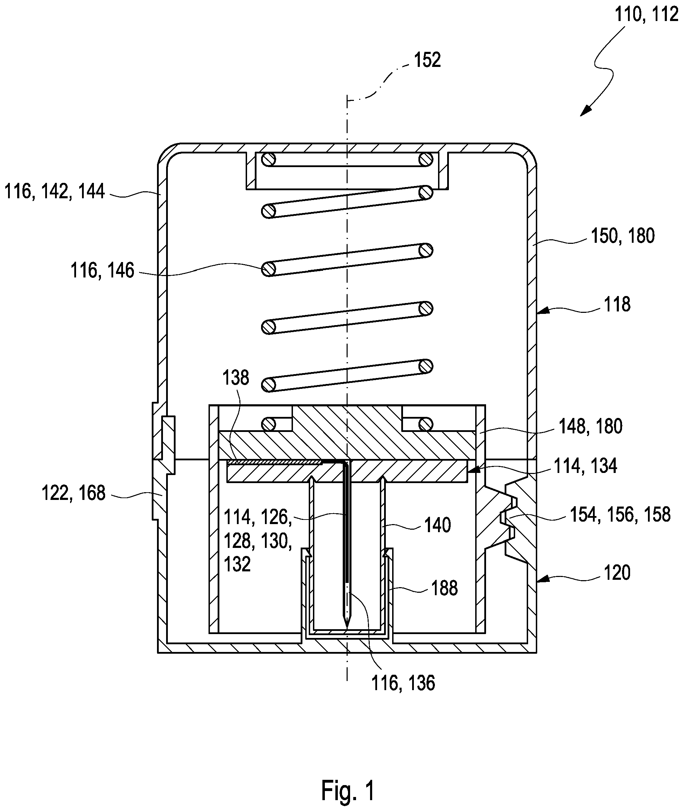

In Figure 1, an exemplary embodiment of an apparatus 110 is shown in a cross-sectional view. The apparatus 110 may specifically be a medical system 112. The apparatus 110, specifically the medical system 112, comprises:
a. a medical device 114 which is at least partially insertable into a body tissue of a user;
b. an insertion device 116 for at least partially inserting the medical device 114 into the body tissue;
c. a housing 118; and
d. a removable cap 120 connected to the housing 118, the removable cap 120 being configured for being removed from the housing 118 before insertion of the medical device 114.

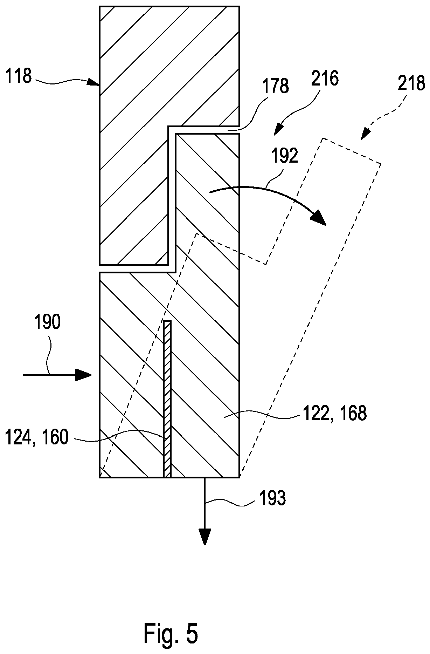

The housing 118 and the removable cap 120, prior to removal of the removable cap 120, are connected via at least one flexible connection element 122. The flexible connection element 122 is reversibly displaceable from a locking position in which the removable cap 120 is secured to the housing 118 by the flexible connection element 122 into a releasing position in which the removable cap 120 is released. An example of the locking position and the releasing position will be given with respect to Figure 5 below. The flexible connection element 122, prior to use, is secured in the locking position by at least one frangible securing element 124. The frangible securing element 124 is not visible in Figure 1. An exemplary embodiment of the frangible securing element 124 is shown, for example, in Figures 5 to 7.

The apparatus 110 may comprise at least one element configured for performing at least one medical function and/or for being used in at least one medical process. For example, the apparatus 110 comprises the medical device 114 which may be configured for performing the at least one medical function and/or for being used in the medical process. Further, the apparatus 110 may comprise components which may provide an insertion mechanism, such as components configured for inserting the medical device 114 into the body tissue of the user. For example, the apparatus 110 comprises the at least one insertion device 116 for at least partially inserting the medical device 114 into the body tissue. The apparatus 110 specifically may be configured for performing at least one of the following actions: the action of inserting the medical device 114 into the body tissue; the action of detecting at least one analyte in the body fluid by using an analyte sensor 128; the action of delivering of at least one therapeutic fluid into the body tissue.

The medical device 114 may comprise at least one of a device 126 for delivery of the at least one therapeutic fluid and an analyte sensor 128 for detecting the at least one analyte. Specifically, the device 126 for delivery of the therapeutic fluid may be or may comprise at least one infusion set 130 comprising at least one infusion cannula 132. The analyte sensor 128 may be an analyte sensor 128 for detecting at least one bodily analyte in at least one bodily fluid present in the body tissue of the user. More specifically, the analyte sensor 128 may be at least one electrochemical analyte sensor comprising at least one sensor electrode for detecting the at least one analyte.

The medical device 114 may be at least partially accommodated within the housing 118. The medical device 114 may be comprised by at least one preassembled functional module 134 of the apparatus 110, specifically by at least one penetrating cartridge. The preassembled functional module 134 may comprise, besides the medical device 114, specifically besides the analyte sensor 128, at least one of the following components: at least one penetrating member 136 for inserting the medical device 114 into the body tissue, more specifically at least one insertion needle; the at least one infusion cannula 132 for delivery of at least one therapeutic fluid into a body tissue of a user; at least one electronics unit 138 electrically connected to the medical device 114, specifically at least one electronics unit 138 for sensing the at least one analyte in conjunction with the at least one analyte sensor 128. The medical device 114 may comprise at least one portion which is at least one of transcutaneously or subcutaneously insertable into the body tissue.

The insertion device 116 may be configured for moving the preassembled functional module 134 towards the skin of the user, specifically resulting in the at least partial insertion of the medical device 114 into the body tissue. The preassembled functional module 134 may further comprise at least one protective cap 140, specifically at least one sterility cap. The protective cap 140 may provide a sterile surround for the medical device 114 and/or the penetrating member 136. The apparatus 110 may be configured such that removing the removable cap 120 removes the protective cap 140 from the preassembled functional module 134, specifically by twist-off.

The insertion device 116 may comprise at least one actuator 142 for driving the medical device 114 into the body tissue of the user. Specifically, the insertion device 116 may comprise at least one actuable displacement mechanism 144 which upon actuation is capable of driving and/or protracting the medical device 114 towards the body tissue of the user. The driving and/or protraction may result in the at least partial insertion of the medical device 114 into the body tissue of the user. The actuator 142 may be a manual actuator 142 which is manually actuable by the user.

Further, the actuator 142 may comprise at least one of the following: an insertion spring for driving the medical device 114 into the body tissue of the user; a return spring 146 for retracting the penetrating member 136 from the body tissue after insertion, specifically by leaving the medical device 114 in place. The insertion spring is not visible in the apparatus 110 shown in Figure 1. The return spring 146 may be configured for retracting the penetrating member 136 from the body tissue of the user, specifically after insertion of the medical device 114.

The penetrating member 136 may be part of the apparatus 110, specifically of the insertion device 116. The penetrating member 136 may be configured for inserting the medical device 114 into the body tissue. When retracting the penetrating member 136, for example by using the return spring 146, the penetrating member 136 may be configured for leaving the medical device 114 in the body tissue.

The insertion device 116, specifically the actuator 142, may comprise at least one part of the housing 118. The housing 118 may comprise at least one stationary portion 148 configured for resting on the skin of the user. The housing 118 may further comprise at least one actuation portion 150 configured for being manually actuated by the user for actuating the actuator 142. The stationary portion 148 and the actuation portion 150 may be movable with respect to each other. The stationary portion 148 and the actuation portion 150 each may comprise cylindrical parts which are concentrically disposed with respect to a longitudinal axis 152 of the apparatus 110. The housing 118 may be at least partially cylindrical with respect to the longitudinal axis 152 of the apparatus 110.

The removable cap 120 may be connected to the housing 118 by a twistable connection 154, specifically by at least one screw coupling 156. At least one of the removable cap 120 and the housing 118 may comprise at least one screw thread 158. Specifically, both of the housing 118 and the removable cap 120 may comprise screw threads 158 corresponding to each other, such that the screw threads 158 may form the twistable connection 154 between the housing 118 and the removable cap 120. The removable cap 120 may be at least partially cylindrical. Specifically, the removable cap 120 may be located concentrically with respect to the longitudinal axis 152 of the apparatus 110.

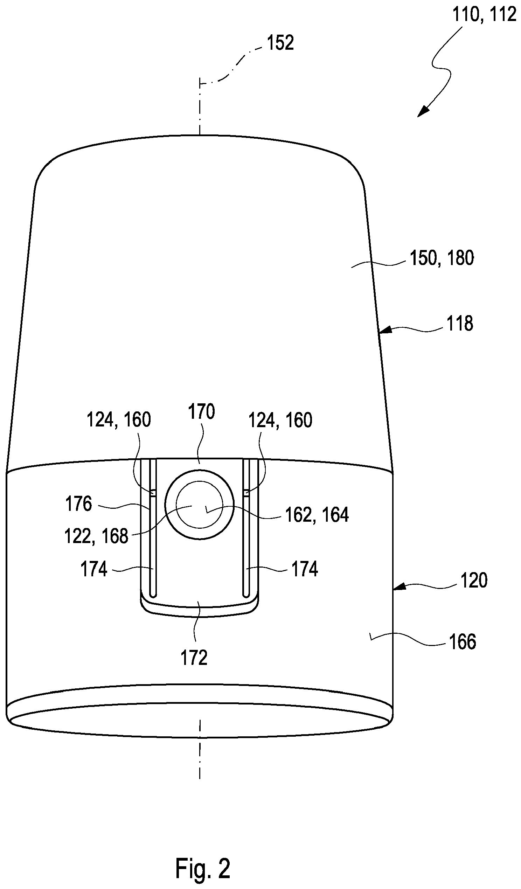

The flexible connection element 122 may be at least partially integrated into one of the removable cap 120 and the housing 118. The flexible connection element 122 will be described in further detail below, with reference to Figures 2 and 5 to 7.

In Figures 2 to 4, several perspective views of the apparatus 110 are shown which will be explained in further detail and in conjunction below. Therein, the interior of the apparatus 110 may be embodied as explained above in the context of Figure 1. It shall be noted, however, that other embodiments of the apparatus 110 are also feasible in conjunction with Figures 2 to 4, such as other embodiments of the medical device 114 and/or of the insertion device 116.

Therein, Figure 2 shows an exemplary embodiment of the apparatus 110 with the removable cap 120 connected to the housing 118 in a perspective view. The housing 118 and the removable cap 120 are connected via the at least one flexible connection element 122. Specifically, the housing 118 and the removable cap 120, prior to removal of the removable cap 120, may be locked to each other by at least one of a force-fit or a form-fit connection. As in Figure 1, the removable cap 120 may be connected to the housing 118 by a twistable connection 154, which is not visible in Figure 2.

As outlined above, the flexible connection element 122 is reversibly displaceable from the locking position in which the removable cap 120 is secured to the housing 118 by the flexible connection element 122 into the releasing position in which the removable cap 120 is released. The flexible connection element 122, prior to use, is secured in the locking position by the at least one frangible securing element 124. In Figure 2, the flexible connection element 122 is in the locking position and, thus, the removable cap 120 is secured to the housing 118.

The frangible securing element 124 may comprise at least one frangible ligament 160 securing at least part of the flexible connection element 122 in the locking position. Specifically, the frangible securing element 124 may secure the flexible connection element 122 in the locking position to the removable cap 120. The flexible connection element 122 may be movable form the locking position into the releasing position The frangible securing element 124 may be configured for being destroyed when the flexible connection element 122 is moved from the locking position into the releasing position. A direction of motion of the flexible connection element 122 from the locking position into the releasing position may be different from a direction of a force required for removing the removable cap 120 from the housing 118. As can be seen in Figure 2, the apparatus 110, specifically the housing 118 and the removable cap 120, may have a cylindrical shape and, thus, may extend along the longitudinal axis 152. The direction of motion of the flexible connection element 122 from the locking position into the releasing position may be a radial direction with respect to the longitudinal axis 152 of the apparatus 110. The direction of force required for removing the cap may comprise a direction perpendicular to the radial direction, such as a longitudinal direction with respect to the longitudinal axis 152 or a rotational direction about the longitudinal axis 152. The flexible connection element 122 may be configured, due to its elastic properties, for returning from the releasing position into the locking position by an elastic force.

The flexible connection element 122 may comprise at least one force application surface 162 accessible to the user. A manual force may be applicable via the force application surface 162. Specifically, the force application surface 162 may comprise an indentation 164 in an outer surface 166 of the apparatus 110. The indentation 164 may be configured for receiving a finger tip of the user. The flexible connection element 122 may be flexibly displaceable by at least one of manually pressing, twisting or pulling the flexible connection element 122.

Further, the flexible connection element 122 may be at least partially integrated into one of the removable cap 120 and the housing 118. In the exemplary embodiment shown in Figure 2, the flexible connection element 122 may be integrated into the removable cap 120. The flexible connection element 122 may further be configured for remaining with the removable cap 120 or the housing 118, respectively, after removing the removable cap 120 from the housing 118.

In the exemplary embodiment of Figure 2, the flexible connection element 122 may comprise at least one flexible tongue 168. The flexible tongue 168 may comprise at least one free end 170 and at least one fixed end 172. The fixed end 172 may be connected to one of the housing 118 and the removable cap 120. The free end 170 may be configured for engaging with at least one of the housing 118 and the removable cap 120. The frangible securing element 124 may secure the flexible tongue 168 in the locking position.

The flexible tongue 168 may be at least partially received within a slot 174 of one of the housing 118 and the removable cap 120. The frangible securing element 124 may secure the flexible tongue 168 to at least one edge 176 of the slot 174. As can be seen in Figure 2, the flexible tongue 168 may, as an example, be received within the slot 174 of the removable cap 120.

Specifically, the flexible connection element 122 may be part of the removable cap 120 and may be configured to engage with housing 118 in the locking position. The flexible connection element 122 may be flexibly displaceable into the releasing position by disengaging with the housing 118.

The apparatus 110 may be configured such that bringing the flexible connection element 122 from the locking position into the releasing position and removing of the removable cap 120 from the housing 118 may require separate mechanical actions by the user. Specifically, the removable cap 120 may be configured such that removing the removable cap 120 from the housing 118 may require a twisting motion of the removable cap 120, specifically a twisting motion of the removable cap 120 about the longitudinal axis 152 of the apparatus 110. Additionally, bringing the flexible connection element 122 from the locking position into the releasing position may require a mechanical action of applying the manual force to the flexible connection element 122, such as by manually pressing, twisting or pulling the flexible connection element 122.

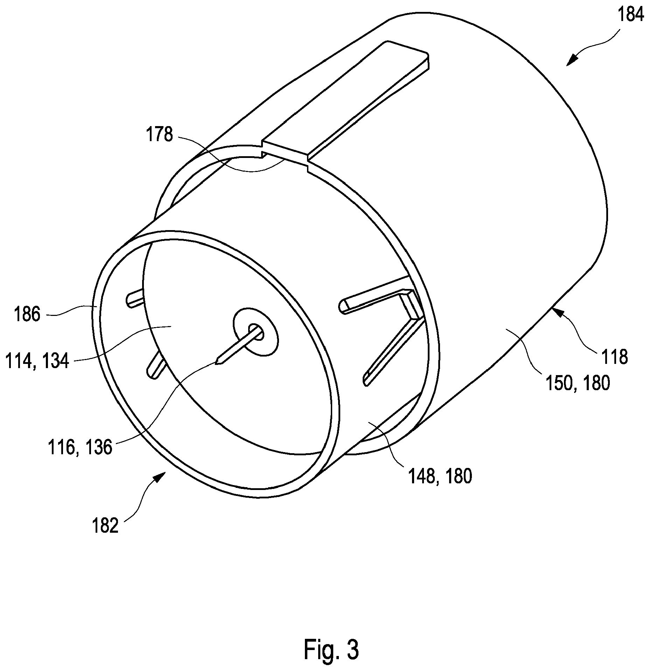

Figure 3 shows an exemplary embodiment of the apparatus 110 without the removable cap 120 in a perspective view. As can be seen in Figure 3, the removable cap 120 has been removed from the housing 118 of the apparatus 110. The housing 118 of the apparatus 110 may comprise the actuation portion 150 at least partially surrounding the stationary portion 148 of the housing 118. The medical device 114 may be comprised by the preassembled functional module 134. The preassembled functional module 134 may be received within the stationary portion 148 of the housing 118. The preassembled functional module 134 may further comprise the penetrating member 136. The penetrating member 136 may be part of the apparatus 110, specifically of the insertion device 116. The penetrating member 136 may protrude from the preassembled functional module 134, specifically pointing towards the skin of the user.

As outlined above, the flexible connection element 122 may be connected to, specifically integrated into, one of the housing 118 and the removable cap 120. In the exemplary embodiment shown in Figure 2, the flexible connection element 122 may be connected to the removable cap 120 and may remain with the removable cap 120 after removing the removable cap 120 from the housing 118. Further, the other one of the housing 118 and the removable cap 120 may comprise at least one depression 178. In the exemplary embodiment shown in Figure 3, the housing 118 may comprise the depression 178. The depression 178 may be configured for engaging with the flexible connection element 122 in the locking position.

The housing 118 may further comprise at least one sheath 180 with a distal side 182 facing the skin of the user and a proximal side 184 facing away from the user. The removable cap 120 may cover at least the distal side 182 of the housing 118 prior to placement of the apparatus 110 onto the skin of the user, specifically prior to removal of the removable cap 120. The insertion device 116 may be configured for placement onto the skin of the user, specifically by a circumferential rim 186 of the housing 118.

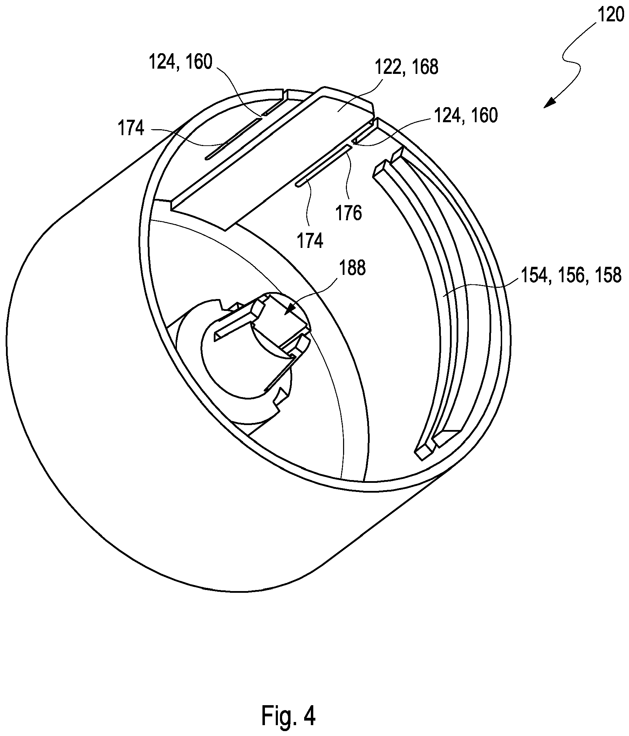

In Figure 4, an exemplary embodiment of the removable cap 120 is shown in a perspective view. The removable cap 120 may be configured such that removing the removable cap 120 from the housing 118 may require the twisting motion. For example, the removable cap 120 may comprise the at least one screw thread 158. The removable cap 120 may be connected to the housing 118 by the twistable connection 154, specifically by at least one screw coupling 156.

In the exemplary embodiment shown in Figure 4, the flexible connection element 122 may be at least partially integrated into the removable cap 120. The flexible connection element 122 may comprise the at least one flexible tongue 168. The removable cap 120 may comprise the at least one slot 174, wherein the slot 174 may be configured for at least partially receiving the flexible tongue 168. The frangible securing element 124 may secure the flexible tongue 168 to the at least one edge 176 of the slot 174.

As outlined above in the context of Figure 1, the preassembled functional module 134 may comprise the at least one protective cap 140. The preassembled functional module 134 with the protective cap 140 may be received within the housing 118 and, thus, is not visible in Figure 4. The removable cap 120 may comprise at least one inner contour 188. The inner contour 188 of the removable cap 120 may be configured for engaging with an outer contour of the protective cap 140.

The removable cap 120 may be configured for being removed from the housing 118 by a rotational movement. The removable cap 120 may further be configured for transferring a torque onto the protective cap 140 during removal. The protective cap 140 may be removed from the preassembled functional module 134 by the torque transferred from the inner contour 188 of the removable cap 120 to the outer contour of the protective cap 140.

The apparatus 110 may further comprise at least one removable protective cap, specifically at least one removable protective cap connected to the preassembled functional module 134. The cylindrical removable cap may be configured for removing the removable protective cap when being removed from the housing 118. The cylindrical removable cap may comprise at least one engaging contour for engaging with a corresponding contour of the removable protective cap.

Figures 5 to 7 show various embodiments of the flexible connection element 122 in a cross-sectional view. These embodiments may be implemented, as an example, in the embodiments of the apparatus 110 as shown above. It shall be noted, however, that other embodiments of the apparatus 110 are also feasible.

Therein, Figure 5 shows a first exemplary embodiment of the flexible connection element 122 in a cross-sectional view. As outlined above, the apparatus 110 comprises the housing 118 and the removable cap 120, wherein the housing 118 and the removable cap 120, prior to removal of the removable cap 120, are connected via the flexible connection element 122. In the exemplary embodiment shown in Figure 5, the flexible connection element 122 may be integrated into the removable cap 120, which is not visible in Figure 5. The frangible securing element 124 may secure the flexible connection element 122 to the removable cap 120.

The flexible connection element 122 shown in solid lines in Figure 5 is in the locking position, as denoted symbolically by reference number 216. Generally, the locking position 216 may also be referred to as a "locking configuration". The flexible connection element 122 may be received within the at least one depression 178 of one of the removable cap 120 and the housing 118. In the exemplary embodiment shown in Figure 5, in the locking position 216, the flexible connection element 122 may be received in the depression 178 of the housing 118. The frangible securing element 124 may secure the flexible connection element 122 in the locking position 216. Specifically, the frangible securing element 124 may prevent movement of the flexible connection element 122.

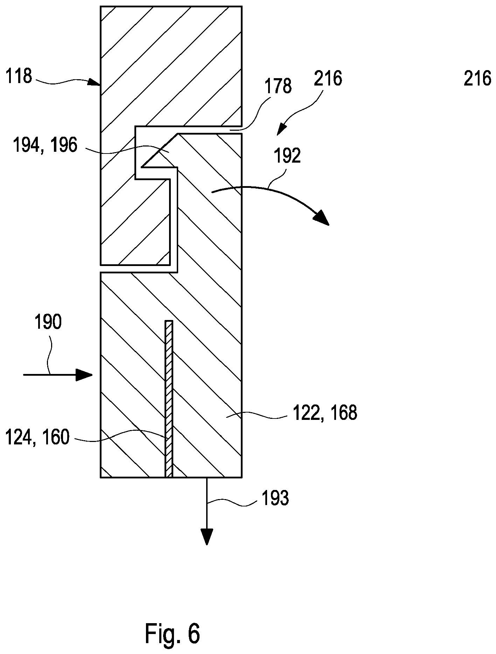

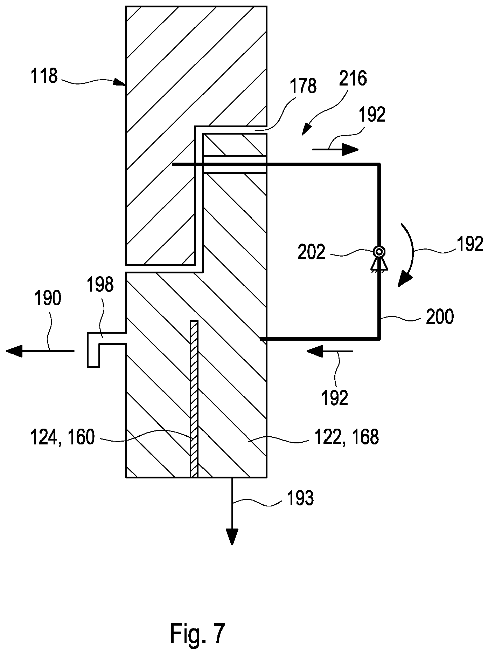

As outlined above, the flexible connection element 122 may be movable from the locking position 216 into the releasing position by applying the manual force. In Figure 5, the manual force is symbolically denoted by arrow 190. The releasing position of the flexible connection element 122, which generally also may be referred to as a "releasing configuration", is indicated by dashed lines and by symbolic reference number 218. It shall be noted that, in the following embodiments of Figures 6 and 7, the releasing configuration is not depicted. The skilled person, however, will recognize that the releasing configuration may be embodied analogously to the embodiment of Figure 5.

The flexible connection element 122 may be movable, upon application of the manual force 190, back and forth in a direction perpendicular to the longitudinal axis 152, as depicted in Figures 1 and 2, of the apparatus 110, specifically into the releasing position 218. The manual force 190 may be applied by the user of the apparatus 110. The frangible securing element 124 may be configured for being destroyed by the manual force 190. A direction of the manual force 190 may be different from a direction of a force required for removing the removable cap 120 from the housing 118.

The flexible connection element 122 may be movable from the locking position 216 into the releasing position 218 in a releasing direction 192. The releasing direction 192 is indicated in Figure 5 by a bent arrow pointing in the direction of releasing, wherein the curvature of the arrow may follow the trajectory of an arbitrary point or element of the flexible connection element 122 during movement. For example, the releasing direction 192 may essentially be a radial direction with respect to the longitudinal axis 152 of the apparatus 110. The removable cap 120 may be, during removing from the housing 118, movable into at least one removing direction 193. The releasing direction 192 may be different from the removing direction 193. For example, the removing direction 193 may comprise a direction perpendicular to the releasing direction 192, such as a direction parallel to the longitudinal axis 152 of the apparatus 110 or a rotational direction about the longitudinal axis 152.

In case the user applies the manual force 190 to the flexible connection element 122, the frangible securing element 124 may be destroyed. The manual force 190 required to destroy the frangible securing element 124 may be adjustable by a structure and/or a material selection of the frangible securing element 124. When the frangible securing element 124 is destroyed, it may be possible to displace the flexible connection element 122 from the locking position 216 into the releasing position 218, for example by moving the flexible connection element 122 from the locking position 216 into the releasing position 218 and/or by deforming the flexible connection element 122.

In the releasing position 218, the flexible connection element 122 may release the twisting motion of the removable cap 120. In the exemplary embodiment shown in Figure 5, the flexible connection element 122 may disengage with the depression 178 of the housing 118 and, thus, may release a relative motion of the housing 118 and the removable cap 120. In the releasing position 218 of the flexible securing element 122, a rotational motion and/or a translational movement of the removable cap 120 relative to the housing 118 may be possible.

A second exemplary embodiment of the flexible connection element 122 shown in a cross-sectional view in Figure 6 may correspond widely to the first embodiment of the flexible connection element 122 shown in Figure 5. Thus, reference may be made to the description of Figure 5. In the exemplary embodiment shown in Figure 6, the flexible connection element 122 may comprise at least one of a hook 194 or a catch 196. In the locking position 216, additionally or alternatively, the flexible connection element 122 may engage with the depression 178 and prevent a translational motion of the housing 118 and/or the removable cap 120. However, other embodiments, structures or forms may be possible, such as a trapezium-shaped flexible connection element 122 or other geometries of the flexible connection element 122 restricting the translational motion of the housing 118 and/or the removable cap 120.

In the releasing position 218, which is not depicted in Figure 6, the flexible connection element 122 may disengage with the depression 178 in the housing 118. Thus, the flexible connection element 122 may release the translational motion of the housing 118 and/or the removable cap 120 in the releasing position 218. In the exemplary embodiment shown in Figure 6, the flexible connection element 122 may disengage with the depression 178 of the housing 118 and, thus, may release a relative motion of the housing 118 and the removable cap 120.

In Figure 7, a third exemplary embodiment of the flexible connection element 122 is shown in a cross-sectional view. Therein, the flexible connection element 122 may comprise at least one pin 198. Additionally, the apparatus 110 may comprise at least one additional structure 200 configured for indirectly displacing the flexible connection element 122. The additional structure 200 may be configured for inverting the releasing direction 192. The additional structure 200 may further be configured for spatially separating the functionalities of the flexible connection element 122.

In the exemplary embodiment shown in Figure 7, the manual force 190 may comprise pulling the pin 198 of the flexible connection element 122. The frangible securing element 124 may be destroyed by moving the flexible connection element 122 from the locking position 216 into the releasing position 218. In the releasing position 218, a relative movement of the housing 118 and the removable cap 120 may be released. The additional element 200 may be in contact with a pivotal point 202. By the interaction of the additional element 200 and the pivotal point 202, the releasing direction 192 may be inverted. The interlocking of the removable cap 120 and the housing 118 may be released in the releasing position 218.

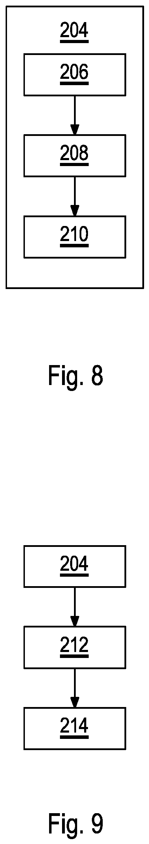

Figure 8 shows a flow chart of an exemplary embodiment of a method for preparing the apparatus 110 for insertion of at least a part of the medical device 114 into the body tissue of the user. The method comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method may comprise additional method steps that are not listed.

The method comprises preparing the apparatus 110 (denoted by reference number 204) according to the present invention, such as according to any one of the embodiments disclosed above and/or any one of the embodiments disclosed in further detail below, for insertion. In a first optional step, the method may comprise providing the apparatus 110 (denoted by reference number 206). The method further comprises displacing the flexible connection element 122 from the locking position 216 into the releasing position 218 (denoted by reference number 208), thereby at least partially destroying the frangible securing element 124. The method further comprises removing the removable cap 120 from the housing 118 (denoted by reference number 210).

In Figure 9, a flow chart of an exemplary embodiment of a method for at least partially inserting at least a part of the medical device 114 of the apparatus 110 into the body tissue of the user is shown. The method comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method may comprise additional method steps that are not listed.

The method comprises at least partially inserting at least part of the medical device 114 of the apparatus 110 according to the present invention, such as according to any one of the embodiments disclosed above and/or according to any one of the embodiments disclosed in further detail below. The method comprises preparing the apparatus 110 for insertion (denoted by reference number 204) by using the method according to present invention, such as according to any one of the embodiments disclosed above and/or according to any one of the embodiments disclosed in further detail below. The method further comprises placing a distal end of the housing 118 onto the skin of the user (denoted by reference number 212) and actuating the insertion device 116 (denoted by reference number 214).

### List of reference numbers

- 110: apparatus
- 112: medical system
- 114: medical device
- 116: insertion device
- 118: housing
- 120: removable cap
- 122: flexible connection element
- 124: frangible securing element
- 126: device for delivery of a therapeutic fluid
- 128: analyte sensor
- 130: infusion set
- 132: infusion cannula
- 134: preassembled functional module
- 136: penetrating member
- 138: electronics unit
- 140: protective cap
- 142: actuator
- 144: actuable displacement mechanism
- 146: return spring
- 148: stationary portion
- 150: actuation portion
- 152: longitudinal axis
- 154: twistable connection
- 156: screw coupling
- 158: screw thread
- 160: frangible ligament
- 162: force application surface
- 164: indentation
- 166: outer surface
- 168: flexible tongue
- 170: free end
- 172: fixed end
- 174: slot
- 176: edge of the slot
- 178: depression
- 180: sheath
- 182: distal side
- 184: proximal side
- 186: circumferential rim
- 188: inner contour
- 190: manual force
- 192: releasing direction
- 193: removing direction
- 194: hook
- 196: catch
- 198: pin
- 200: additional element
- 202: pivotal point
- 204: preparing the apparatus for insertion
- 206: providing the apparatus
- 208: displacing the flexible connection element
- 210: removing the removable cap
- 212: placing a distal end of the housing onto the skin of the user
- 214: actuating the insertion device
- 216: locking position
- 218: releasing position

## Claims

1. An apparatus (110) comprising:
a. a medical device (114) which is at least partially insertable into a body tissue of a user;
b. an insertion device (116) for at least partially inserting the medical device (114) into the body tissue;
c. a housing (118); and
d. a removable cap (120) connected to the housing (118), the removable cap (120) being configured for being removed from the housing (118) before insertion of the medical device (114),
wherein the housing (118) and the removable cap (120), prior to removal of the removable cap (120), are connected via at least one flexible connection element (122), wherein the flexible connection element (122) is reversibly displaceable from a locking position (216) in which the removable cap (120) is secured to the housing (118) by the flexible connection element (122) into a releasing position (218) in which the removable cap (120) is released, wherein the flexible connection element (122), prior to use, is secured in the locking position (216) by at least one frangible securing element (124).

2. The apparatus (110) according to any one of the preceding claims, wherein the flexible connection element (122) is movable from the locking position (216) into the releasing position (218) by applying a manual force (190), wherein the frangible securing element (124) is configured for being destroyed by the manual force (190), wherein a direction of the manual force (190) is different from a direction of a force required for removing the removable cap (120) from the housing (118).

3. The apparatus (110) according to any one of the preceding claims, wherein the flexible connection element (122) is at least partially integrated into one of the removable cap (120) and the housing (118), wherein the flexible connection element (122) is further configured for remaining with the removable cap (120) or the housing (118), respectively, after removing the removable cap (120) from the housing (118).

4. The apparatus (110) according to any one of the preceding claims, wherein the flexible connection element (122) is flexibly displaceable by at least one of manually pressing, twisting or pulling the flexible connection element (122).

5. The apparatus (110) according to any one of the preceding claims, wherein the flexible connection element (122) comprises at least one of a flexible tongue (168), a hook (194), a catch (196) or a pin (198).

6. The apparatus (110) according to any one of the preceding claims, wherein the flexible connection element (122) comprises at least one flexible tongue (168), wherein the flexible tongue (168) comprises at least one free end (170) and at least one fixed end (172), the fixed end (172) being connected to one of the housing (118) and the removable cap (120), wherein the frangible securing element (124) secures the flexible tongue (168) in the locking position (216).

7. The apparatus (110) according to any one of the preceding claims, wherein the flexible connection element (122) is movable from the locking position (216) into the releasing position (218) in a releasing direction (192), wherein the removable cap (120) is, during removing from the housing (118), movable into at least one removing direction (193), wherein the releasing direction (192) is different from the removing direction (193).

8. The apparatus (110) according to any one of the preceding claims, wherein the apparatus (110) is configured such that bringing the flexible connection element (122) from the locking position (216) into the releasing position (218) and removing of the removable cap (120) from the housing (118) requires separate mechanical actions by the user.

9. The apparatus (110) according to any one of the preceding claims, wherein the removable cap (120) is configured such that removing the removable cap (120) from the housing (118) requires a twisting motion of the removable cap (120).

10. The apparatus (110) according to any one of the preceding claims, wherein the medical device (114) comprises at least one of a device (126) for delivery of at least one therapeutic fluid into a body tissue of a user, specifically at least one infusion set (130) comprising at least one infusion cannula (132); at least one analyte sensor (128) for detecting at least one analyte; specifically at least one analyte sensor (128) for detecting at least one bodily analyte in the at least one bodily fluid, more specifically at least one electrochemical analyte sensor (128) comprising at least one sensor electrode for detecting the at least one analyte.

11. The apparatus (110) according to any one of the preceding claims, wherein the insertion device (116) comprises at least one actuator (142) for driving the medical device (114) into the body tissue, wherein the insertion device (116) comprises at least one part of the housing (118), wherein the housing (118) comprises at least one stationary portion (148) configured for resting on the skin of the user and at least one actuation portion (150) configured for being manually actuated by the user for actuating the actuator (142), wherein the stationary portion (148) and the actuation portion (150) are movable with respect to each other.

12. The apparatus (110) according to any one of the preceding claims, wherein the medical device (114) is comprised by at least one preassembled functional module (134) of the apparatus (110), the at least one preassembled functional module (134) comprising, besides the at least one medical device (114), at least one of the following components: at least one penetrating member (136) for inserting the medical device (114) into the body tissue; at least one infusion cannula for delivery of at least one therapeutic fluid into a body tissue of a user; at least one electronics unit (138) electrically connected to the medical device (114).

13. The apparatus (110) according to the preceding claim, wherein the preassembled functional module (134) comprises at least one protective cap (140), wherein the apparatus (110) is configured such that removing the removable cap (120) removes the protective cap (140) from the preassembled functional module (134).

14. A method for preparing the apparatus (110) according to any one of the preceding claims for insertion of at least a part of the medical device (114) into a body tissue of a user, the method comprising displacing the flexible connection element (122) from the locking position (216) into the releasing position (218), thereby at least partially destroying the frangible securing element (124), the method further comprising removing the removable cap (120) from the housing (118).

15. A method for inserting at least a part of the medical device (114) of the apparatus (110) according to any one of the preceding claims referring to an apparatus (110) into a body tissue of a user, the method comprising preparing the apparatus (110) for insertion by using the method according to the preceding claim, the method further comprising placing a distal end of the housing (118) onto the skin of the user and actuating the insertion device (116).
